# EUROPEAN PATENT APPLICATION

(11) **EP 4 814 983 A1**
(43) Date of publication of application: **30.09.2026**
(21) Application number: 25166949.5
(22) Date of filing: 28.03.2025
(51) Int. Cl.: G16H 50/20, G16H 50/30, G16H 50/70, A61B 5/01, A61B 5/02, A61B 5/08

(54) **METHOD FOR MONITORING THE HEALTH STATUS OF A PATIENT**

(71) Applicant: Capsula S.r.l., 20121 Milano (MI) (IT)
(72) Inventor: Nizardo Chailly, Alessandro, 20121 Milano MI (IT); Andreoni, Giuseppe, 20835 Muggiò MB (IT)
(74) Representative: Perani & Partners S.p.A.

(57) **Abstract**

The invention concerns a method for monitoring the health status of a patient, preferably by means of a physical-digital system, comprising the steps of a) detecting a plurality of patient data by means of a plurality of biomedical sensors, b) storing the plurality of patient data in a current database, c) generating a digital health twin of at least a patient by means the elaboration of the plurality of patient data for a specific patient through an elaboration unit in signal communication with the current database. The method further comprises d) providing a predictive artificial intelligence and a historical database representative of the correlation between the health status of a patient and his physiological signals and parameters, and training the predictive algorithm with said historical database, the artificial intelligence algorithm being trained with machine learning systems in order to extract a set of most relevant features from the digital health twin, and e) estimating the health status of the patient in function of the set of relevant features extracted.

## Description

### FIELD OF APPLICATION

The object of the present invention is used in the field of digital healthcare, with particular attention to the area of lifestyle medicine and the prevention of chronic diseases.

### State of the prior art

Different systems and methods for monitoring the health status of a patient are known in the state of art. We refer to automated systems for check-ups known as health pods, i.e. small spaces equipped with a set of biomedical devices to measure some parameters related to human health. Generally, health pods can offer nurse-assisted or autonomous and customized health evaluations by means of biomedical sensors and digital platforms. These technologies allow to collect data about the health status of a patient in a dedicated measurement sessions way. The state-of-the-art analysis emphasises the versatility of sensors, user protection, device interoperability and the central role of the Internet of Things (IoT), which improves the interconnection between devices.

### Problem of the prior art

The known system and methods show a list of unresolved issues:
They represent a special "sum of technology" without an actual integration, where a multidevice box is included in the system and a nurse assists in the different measurements; a dedicated close and "personal" space is also missing.

In the frame of the P4 medicine approach (preventive, predictive, personalized and participatory) as suggested by EU vision there is the need of the user's direct participation in the process: this means to create an intuitive process that is capable to drive the user in the complete process and avoiding errors in procedures and have intuitive usability.

Another negative aspect of known methods and systems is to provide a clear understanding of the measured health status and clear message on what to do at the end of the measurements, eventually giving the possibility to activate specific services.. Currently, known technologies don't have neither ergonomic and intuitive interface in order to simplify user experience nor physical spaces acted to conjugate technology and privacy.

### SUMMARY OF THE INVENTION

The object of the present invention is to provide a method for monitoring health status of a patient able to overcome the drawbacks of the prior art.

A further object of the present invention is to make available a method able to offer a holistic approach for the health management.

This and further objects will be substantially achieved by a method for monitoring the health status of a patient in accordance with one or more of the appended claims.

### Advantages of the invention

Thanks to the present invention, it's possible to obtain a method able to combine autonomous measurements of several quantitative and subjective parameters of user's own health status with their advanced and integrated digital representation in order to offer a complete in-depth study about health status with their advanced and integrated digital representation leverages the concept of personalized digital health twin generating a multidimensional health score.

Advantageously, the method of the present invention introduces a physical-digital ecosystem thus avoiding the need of sanitary specialists and making the systems more accessible and scaled with its flexible architecture and modular design, the system enhances the ability to analyse quantitative parameters and adapt to different healthcare settings.

Further advantageously, the method provides preventive and customized analysis by taking multidimensional correlation into account. Furthermore, thanks to a specific embodiment, the method provides analysis on cluster of subjects who manifest common health features in order to define analysis of prevention, monitoring, screening supervision. The present method enables the computation of health scores allowing for a clinical risk through analyses provided by the integrated kiosk AI system.

The eventually identified risk facilitates the development of targeted and personalized interventions.

The method also support data analysis and presentation in an aggregated form for community intervention, like clusterization in classes of patients/subjects.

### BRIEF DESCRIPTION OF THE DRAWINGS

The advantages and features of the present invention will be amply elucidated by the following detailed description of a preferred embodiment of a method for measuring and assessing and monitoring the health status of a patient, in accordance with the present invention, shown by way of example in the set of drawings of which:
- figure 1 shows a sequence of the steps of a method of the present invention according to a first embodiment,
- figure 2a and figure 2b show an example of a clusterization obtained with the method of the preset invention according to a preferred embodiment.

### DETAILED DESCRIPTION

Object of the present invention is a method for monitoring the health status of a patient, by means of a physical-digital system.

The present method can also evaluate and/or manage pathologies. Pathologies can comprise acute and/or chronic pathologies.

Further, the present method is suitable for for monitoring the health status of a subject for prevention.

It's to be noted that the term "monitoring" can include actions like assessing and/or measuring the health status of a patient.

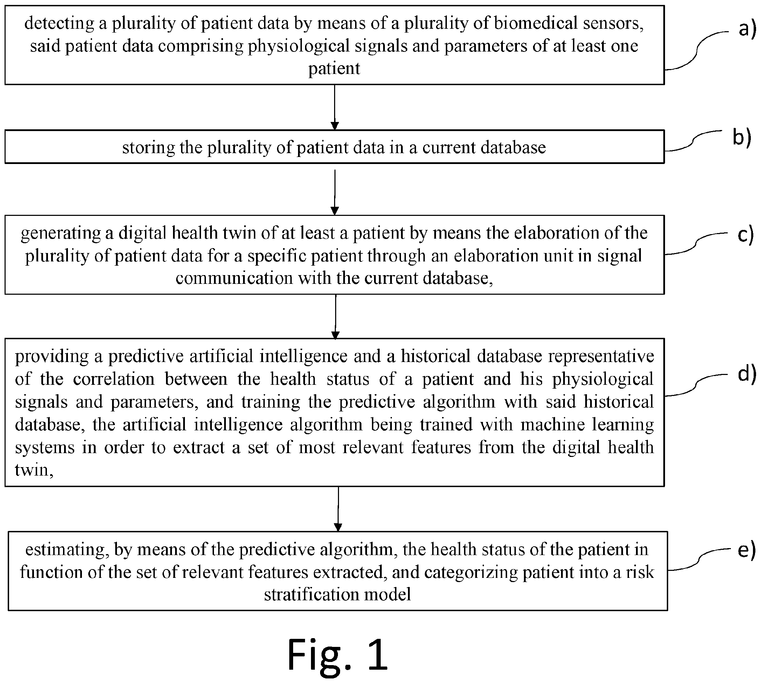

As shown in figure 1, the method comprises the step of a) detecting and, preferably measuring, a plurality of patient data by means of a plurality of biomedical sensors, such as a health pod which includes a plurality of biometric sensors.

The patient data comprise physiological signals and parameters of at least one patient. In a preferred embodiment, physiological signals and parameters comprise a wide set of physiological, functional, lifestyle parameters to be measured preferably by means of non invasive technologies like - but not only - heart rate, ECG traces, weight and body impedance and body composition analysis, body mass index, nutrition, metabolic status, respiratory rate, biological age and blood pressure, hand grip strength, oxygen saturation and body temperature, visual acuity, hearing capability, that can be integrated into a single system in the form of a health pod.

It should be noted that in alternative embodiments only some parameters among the ones previously described can be considered. Again alternatively, it's possible to integrate further signals or parameters in order to obtain, for example, more specific analysis.

The method comprises the further step of b) storing the plurality of patient data in a current database. Preferably, the current database is a structured database.

The method of the present invention comprises the step of c) generating a digital health twin of at least a patient by means the elaboration of the plurality of patient data for a specific patient. The digital health twin, according to the method is obtained through an elaboration unit, preferably a server cloud, which is in signal communication with the current database.

In other words, the processed physiological signals and parameters comprised in the current database are transformed into a dynamic digital representation that serves as a virtual counterpart to the user's overall health status. The digital health twin acts as "digital alter ego" which consolidates physiological metrics and, preferably, lifestyle factors and behavioural trends into a unified picture, thus enabling continuous monitoring and in-depth analysis.

This digital twin can offer a comprehensive analysis by replicating physical, physiological, and metabolic aspects, generating a multidimensional health score for the patient, and enabling a personalized, holistic approach to continuous health monitoring.

Advantageously, the health digital twin confers an integrated and easily interpretable view of the user's health profile.

It's worthwhile noting that, according to the method of the invention, the biomedical sensor and/or the health pod mentioned above are configured to continuously monitor physiological signals and parameters of the patient and update, by means of the elaboration unit, the digital health twin in real time, thereby enabling dynamic interaction between a physical system and the corresponding digital twin to enhance the accuracy of health status prediction.

In particular, the method comprises the step of d) providing a predictive artificial intelligence and a historical database representative of the correlation between the health status of a patient and his physiological signals and parameters and training the predictive algorithm with the historical database.

In other words, the historical database comprises physiological signals and parameters of at least a patient, collected in previous cases, and the correlated to the same signals and parameters stored in the current database to be analysed.

In detail, the artificial intelligence algorithm is trained with machine learning systems in order to extract a set of most relevant features from the digital health twin.

In accordance with the present invention, machine learning systems used for training the predictive algorithm comprise artificial intelligence algorithms chosen among Principal Component Analysis (PCA) Standard Vector Machines (SVM), Dynamically Dimensioned Search (DDS), and/or neural networks with supervised/unsupervised learning able to optimize the extraction of significant information, thus improving predictive analysis.

The most relevant features can be:
- Early signs of possible pathologies;
- Risk factors for the occurrence of possible pathologies in the next future, according to prediction algorithms based on scientific literature findings (cardiovascular, metabolic, respiratory, physical, stress, ...);
- Lifestyle analysis and coaching towards the adoption of preventative habits;
- Clusterization in classes of patients/subjects
- Management of chronic diseases.

These features include cardiovascular parameters, metabolic parameters, respiratory parameters, genetic predisposition to diseases, and lifestyle behavioral factors.

In a preferred embodiment of the present invention, the machine learning systems comprise principal component analysis technique (PCA). Advantageously, through the application of PCA, physiological signals and parameters undergo a linear decomposition process, which allows the most influential and relevant features to be extracted for the digital health twin of the patient. The initial principal components account for most of the data's variance. With the first ten components explaining over 95% of the variance, the model can exclude less informative components, streamlining the analysis without substantial information loss.

This analysis allows for the definition of the belonging of the subject to a specific class of the population (healthy, cardiovascular, obese, and related sub-features) in relation to the parameters and their relevance based on the current dataset originally contains a high number of correlated variables (e.g., heart rate, blood pressure, BMI, respiratory rate, oxygen saturation, genetic predisposition, lifestyle factors).

The method of the invention comprises also the step of e) estimating, by means of the predictive algorithm, the health status of the patient in function of the set of most relevant features extracted.

Based on this set of relevant features, the predictive algorithm can categorize the patient into a risk stratification model.

According to a preferred aspect of the invention, the step of a) detecting a plurality of patient data comprises performing genomic and metabolic analyses. Again preferably, performing genomic and metabolic analyses comprises the step of detecting measurements relative to lipid profile, blood glucose, indices of inflammation and genetic predisposition to disease.

Additionally, the step of a) detecting physiological signals and parameters can comprise the step of collecting data relative to the lifestyle of the patient, such as quality of sleep, level of physical activity and dietary habits, by means the compilation of appropriate questionaries.

According to a preferred embodiment, the step of b) storing the plurality of patient data in a current database comprises the step of storing the current database in a cloud and performing a pre-elaboration of the current database.

In other words, once collected, the current database is securely transmitted to cloud, compliant with GDPR normative, wherein the current database undergoes pre-processing, anonymization, if required, and advanced analysis.

Pre-processing and advanced analysis can comprise a plurality of operations such as, but not limited to, data cleaning, standardization, noise reduction, time series alignments. For example, data cleaning removes inconsistent, incomplete, or erroneous values, such as missing heart rate or unrealistic BMI. Normalization and standardization scale physiological parameters (e.g., heart rate, BMI, blood pressure) for balanced model input. Noise reduction filters out sensor-related artifacts in ECG and respiratory data, improving accuracy. Time-series alignment synchronizes signals recorded at different timestamps for consistent analysis.

Other operations regarding the pre-processing and advanced analysis can comprise feature engineering, which extracts meaningful metrics like heart rate variability (HRV) to enhance health insights. These pre-processing steps ensure high-quality data for accurate health monitoring and prediction. For example, a remote relay management system was tested with ten repetitions to ensure reliability in preparing operational units, while trials with ten users confirmed the effectiveness of integrated filters in reducing noise in ECG and respiratory signals.

In a preferred embodiment, the predictive algorithm is stored in an elaboration unit, like a server cloud. Preferably, the predictive algorithm can be stored in the same cloud wherein the current database is stored.

According to said preferred embodiment, the cloud is configured to have access to standardized sanitary models, based on medical researchers. The step of b) storing the current database in a cloud can comprises the step of correlating plurality of patient data of the current database with said standardized sanitary models.

Preferably, the cloud is also configured to generate and to store the digital health twin of a patient. Since the digital health twin is stored in the server cloud, the patient is able to check whenever he/she wants the health status through his/her digital health twin by accessing the cloud through authentication with a smartphone or a computer device.

By way of example, the health digital twin generated through the elaboration unit can act as virtual model of a patient for monitoring, simulating and optimizing the health outcomes. The physiological signals and parameters and, preferably, data of lifestyle and genomic analysis can be processed, for example by an elaboration unit or server, in order to obtain information about several health domains, such as:
- Physical wellbeing and metabolic health: the analysis includes the level of metabolic aging is based on the in-depth analysis of advanced glycation products (AGEs) closely linked to oxidative stress, chronic inflammation amplifying the onset of chronic diseases and to metabolic decline compromising tissue function and the cardiovascular system. Assessment of muscle strength, lean mass, and muscle tone, key factors in maintaining mobility and preventing sarcopenia and cardiovascular risk. Assessment of metabolic nutritional status, defined as the visual perception of anthropometric evaluations as important indicator of overall individual health status.
- Health: in this domain, it's possible to create a virtual replica of an individual that integrates health data in real time. Health digital twin can help to continuously monitor health, predict future conditions, and optimize medical interventions. This domain performs a physiological check-up to identify cardiovascular (blood pressure, heart rate, hypertension), respiratory (lung capacity, asthma, COPD) and metabolic (prediabetes, dyslipidemia) risks via biomarkers, offering continuous monitoring and preventive support.

- Psychophysical wellbeing: this domain assesses lifestyles by analysing heart rate variability (HRV), a key indicator for monitoring overall well-being, stress level, and physical and mental resilience.
- Genomics: the introduction of the genomics domain into the digital health twin represents a cross-domain shift from the other domains that enables personalization of interventions within the various areas have highlighted the role of genomics in personalizing therapies and predicting health risks. The fusion of genomic data with other personal metrics within the health digital twin enables the execution of targeted treatment modalities and tailored preventive strategies, thus promoting a proactive paradigm in health management. This domain includes genomic assessments develop personalized nutritional and pharmacological plans.

Advantageously, this integration facilitates real-time access to data and provides scalability, ensuring efficient and smooth data management.

Preferably, the historical database further comprises the correlation between the health status, correlated to the physiological signals and parameters, and at least one symptomatology. According to a preferred aspect, the step of e) estimating, by means of the predictive algorithm, comprises providing a symptomatology estimation according with the most relevant features of the health digital twin.

According to an example of the method of the present invention, the biomedical sensors record key physiological parameters such as HRV, blood pressure, oxygen saturation, BMI, respiratory rate, and lifestyle/genetic factors. These data are securely stored in a current database and processed to create a digital health twin-a dynamic virtual model that integrates real-time and historical data for continuous health monitoring and risk assessment.

Next, the historical database stores records of past patients, linking physiological signals to known symptoms. The system compares current patient data with past cases to identify patterns. For example, it detects correlations between hypertension risk factors and symptoms like dizziness, fatigue, and shortness of breath, aiding in early risk assessment.

Then, the machine learning algorithm analyzes the most relevant health features to assess symptom risk. Based on detected patterns, the system predicts potential early symptoms and provides preventive recommendations, such as lifestyle changes, medical evaluations, or personalized interventions, for proactive health management.

In other terms, the predictive algorithm acts as a symptom checker, supporting predictive health monitoring and suggesting possible preventive and corrective actions to improve personal health.

Alternatively, in order to provide a symptomatology estimation one or more Decision Support algorithms can be used. Advantageously, these algorithms can be applied to classify any symptoms and produce a hypothesis of diagnosis (symptom checker) on an individual level.

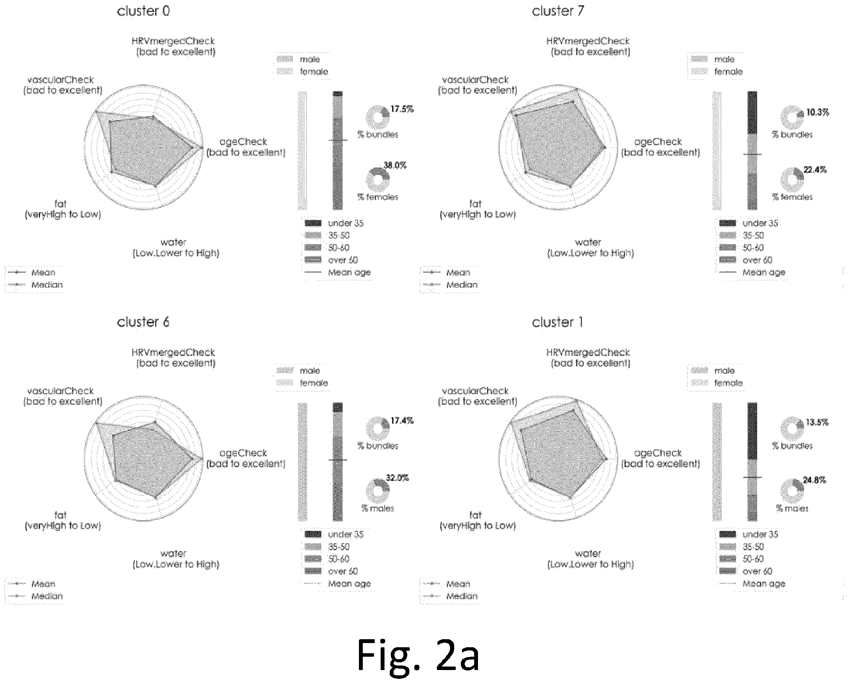

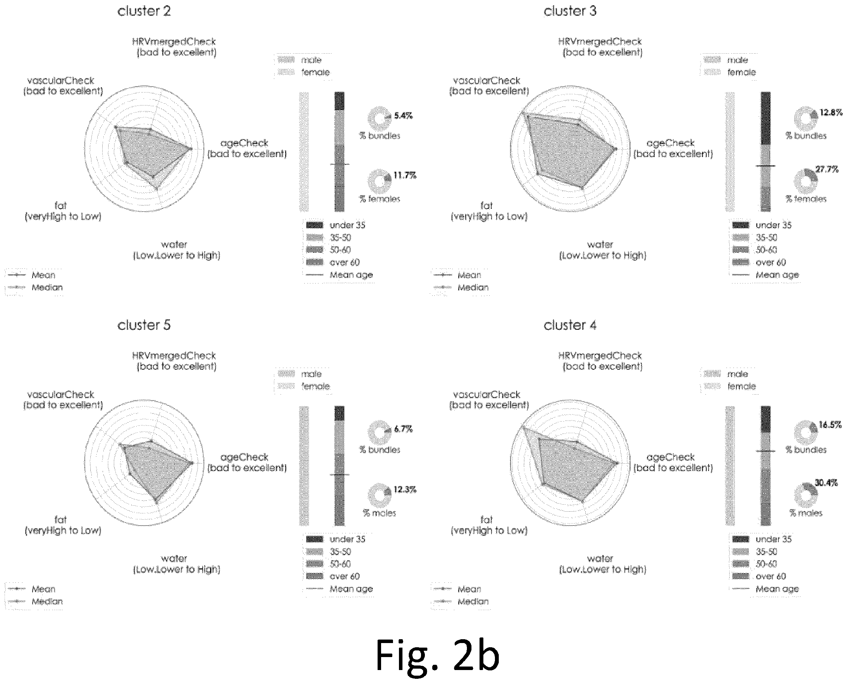

In an embodiment, the predictive algorithm is configured to perform a clusterization of the patients' data collected in the current database in order to obtain group of patients with similar health features. Figure 2a-2b show an example in which, according to an embodiment of the method, the features have been identified. In particular, a use of radar plot for cluster-based analysis of individuals based on physiological and lifestyle parameters is shown in figure 2a-2b. This analysis groups individuals based on their health and lifestyle, showing different risk levels, from high to low, influenced by factors like stress, physical activity, and metabolism. This classification aids in personalized health assessments and preventive recommendations.

More in detail:
- Cluster 1 & Cluster 7 - Balanced Health: Clusters 1 and 7 represent individuals with a generally stable health profile. They have good vascular and metabolic health, with heart rate variability and hydration within normal ranges. These individuals exhibit balanced lifestyle habits, indicating a lower risk of chronic diseases. Risk Level: 4 - Low risk; maintaining current lifestyle habits is recommended.
- Cluster 2 & Cluster 5 - Metabolic Syndrome Risk. Clusters 2 and 5 consist of individuals with metabolic syndrome tendencies, characterized by higher body fat levels, lower vascular health, and potential insulin resistance. These groups face increased risks of obesity, hypertension, and diabetes, requiring nutritional and lifestyle interventions to prevent further health deterioration. Risk Level: 1 - High risk; requires dietary modifications and physical activity.
- Cluster 3 & Cluster 4 - Stressed and Untrained. Clusters 3 and 4 include individuals who are not physically active and experience moderate-to-high stress levels. While they do not necessarily exhibit hypertension, their low cardiovascular resilience and moderate vascular health suggest that exercise and stress management could significantly improve their overall well-being. Risk Level: 3 - Moderate risk; improvements in training and relaxation techniques are advised.

This classification allows for a personalized approach to health monitoring and intervention strategies. The insights from this clustering can help guide preventive health measures and lifestyle modifications to improve health status. To further enhance this personalized approach, a health coach engine can be integrated. The health coach engine leverages clustering insights and the digital health twin's dynamic data to provide real-time, adaptive recommendations tailored to individual's profile.

Preferably, patients with similar health features can join one or more communities, through a dedicated web application, where a community is representative of specific health features. Advantageously, this allows patients to identify common trends or follow-ups based on customized recommendations.

Furthermore, the digital health twin can suggest customized services and resources through a services platform, which is in signal communication with be application through an internet network.

Advantageously, thanks to the interaction among the health pod, the web app for managing results, and a platform for managing epidemiological data the method of the present invention introduces an interactive ecosystem that combines physical devices such as biomedical sensors with digital solutions, thus creating physical-digital (phygital) system and enabling users to take control of their health status in a comprehensive and personalized manner without the need for constant external support thanks to its simple and engaging user experience, users can easily interact with the system, making health management intuitive, accessible, and empowering.

## Claims

1. Method for monitoring the health status of a patient, preferably by means of a physical-digital system, comprising the steps of:
a) detecting a plurality of patient data by means of a plurality of biomedical sensors, said patient data comprising physiological signals and parameters of at least one patient,
b) storing the plurality of patient data in a current database,
**characterized by the steps of**
c) generating a digital health twin of at least a patient by means the elaboration of the plurality of patient data for a specific patient through an elaboration unit in signal communication with the current database,
d) providing a predictive artificial intelligence and a historical database representative of the correlation between the health status of a patient and his physiological signals and parameters, and training the predictive algorithm with said historical database, the artificial intelligence algorithm being trained with machine learning systems in order to extract a set of most relevant features from the digital health twin, machine learning systems comprising artificial intelligence algorithm chosen among Principal Component Analysis, Standard Vector Machines (SVM), Dynamically Dimensioned Search (DDS), and/or neural networks with supervised or unsupervised learning,
e) estimating, by means of the predictive algorithm, the health status of the patient in function of the set of relevant features extracted.

2. Method according with claim 1, wherein physiological signals and parameters comprise heart rate, weight and body impedance, body mass index, heart and respiratory rate, biological age and blood pressure, hand grip strength, oxygen saturation and body temperature.

3. Method according with any of preceding claims, wherein the step of a) detecting a plurality of patient data comprises performing genomic and metabolic analyses.

4. Method according with claim 3, wherein performing genomic and metabolic analyses comprise lipid profile, blood sugar, indices of inflammation, genetic predisposition to diseases.

5. Method according with any of preceding claims, wherein the step of b) storing the plurality of patient data in a database comprises storing database in a cloud and performing a pre-elaboration of the database.

6. Method according with claim 5, wherein the cloud is configured to have access to standardized sanitary models, based on medical researchers, the step of b) storing database in a cloud comprises correlating plurality of patient data of the database with said standardized sanitary models.

7. Method according to any of preceding claims, wherein the historical database further comprises the correlation between the health status, correlated to physiological signals and parameters, and at least one symptomatology, wherein the step pf e) estimating, by means of the predictive algorithm, comprises providing a symptomatology estimation according with the most relevant features of the health digital twin.
